# EUROPÄISCHE PATENTSCHRIFT

(11) **EP 3 402 455 B1**
(45) Veröffentlichungstag und Bekanntmachung des Hinweises auf die Patenterteilung: **20.01.2021**
(21) Anmeldenummer: 16804803.1
(22) Anmeldetag: 01.12.2016
(51) Int. Cl.: A61K 8/06, A61K 8/41, A61Q 17/04

(54) **KOSMETISCHE ZUBEREITUNGEN ENTHALTEND BENZETHONIUM CHLORID UND DIOLE**
COSMETIC COMPOSITIONS CONTAINING BENZETHONIUM-CHLORIDE AND DIOLS
COMPOSITIONS COSMETIQUE CONTENANT CHLORURE DE BENZÉTHONIUM ET UN DIOL

(30) Priorität: 15.01.2016 DE 102016000277
(43) Veröffentlichungstag der Anmeldung: 21.11.2018
(73) Patentinhaber: Beiersdorf AG, 20253 Hamburg (DE)
(72) Erfinder: MEIRING, Uta, 21077 Hamburg (DE); WISCHHÖFER, Svea, 20253 Hamburg (DE); BLECKMANN, Andreas, 22926 Ahrensburg (DE); WAGNER, Antonia, 25421 Pinneberg (DE)
(86) Internationale Anmeldenummer: PCT/EP2016/079377
(87) Internationale Veröffentlichungsnummer: WO 2017/121538

(56) Entgegenhaltungen:
- WO-A1-2011/002929
- WO-A1-2012/019789
- WO-A1-2014/139757
- WO-A2-2012/084410
- WO-A2-2013/143866
- DE-U1- 20 221 386

## Beschreibung

Kosmetische Mittel müssen haltbar sein. Laut Verordnung (EG) Nr. 12239 für die EU bzw. Verordnung über kosmetische Mittel für Deutschland müssen kosmetische Mittel ein Mindesthaltbarkeitsdatum tragen, wenn sie nicht länger als 30 Monate haltbar sind. Das Mindesthaltbarkeitsdatum kann in Form von Text "Mindestens haltbar bis" oder in Symbolform, einer Eieruhr mit Angabe des Datums, auf der Verpackung angebracht sein. Darüber hinaus gibt es eine Kennzeichnung der Haltbarkeit nach Öffnung, nämlich einen geöffneten Cremetiegel mit einer Monatsangabe. Diese Angabe sagt aus, dass das entsprechende Produkt nach dem Öffnen der Verpackung mindestens die angegebene Zeitspanne verwendet werden kann.

Um zu gewährleisten, dass kosmetische Zubereitungen diese Auflagen erfüllen, müssen in der Regel Stoffe zugesetzt werden, die eine konservierende Wirkung haben. Eine konservierende Wirkung bedeutet in erster Linie, dass Keime in ihrem Wachstum (Vermehrung) gehemmt werden und/oder dass Keime abgetötet werden. Als Keime sind in erster Linie Bakterien und Pilze zu nennen.

Kosmetische Zubereitungen werden unter hygienischen Bedingungen hergestellt, d.h. es sind in der Regel keine oder nur wenige Keime in den Zubereitungen enthalten. Wenn beispielsweise Cremetiegel angebrochen werden, können Keime über die Luft oder auch von den Händen der Anwender in die Zubereitung gelangen. Je nach Art des Keimes kann es dann an der Oberfläche der Zubereitung in Anwesenheit von Luft und Nährstoffen aus den Inhaltsstoffen der Zubereitung zu einem Keimwachstum, d.h. zu einer Verkeimung, kommen.

Für kosmetische Zubereitungen gibt es für die EU eine Positivliste an Konservierungsstoffen Verordnung (EG) Nr. 12239, Anhang V (Liste der in kosmetischen Mitteln zugelassenen Konservierungsstoffe), in der aufgeführt ist, welche Substanzen in welchen Höchstmengen in kosmetischen Zubereitungen zugelassen sind. Dies ist eine sinnvolle Einschränkung, um die Gesundheit und das Wohlbefinden der Verbraucher zu gewährleisten. Darüber hinaus gibt es jedoch weitere Studien, die Konservierungsstoffe, die für Kosmetika zugelassen sind, in eine mögliche Verbindung mit Allergien oder möglichen Einflüssen auf das Hormonsystem bringen, siehe dazu beispielsweise Opinion on Parabens, SCCS 1514/13 und Opinion on Methylisothiazolinone (P94) Submission II (Sensitisation only).

Um nun eine ausreichende Konservierung von kosmetischen Zubereitungen zu erzielen, dennoch gleichzeitig die Mengen der Konservierungsstoffe so gering wie möglich zu halten, wird in vielen Fällen mehr als ein Konservierungsmittel eingesetzt, weil verschiedene Konservierungsmittelklassen sich ergänzen oder es sogar zu synergistischen Wirkungen kommt (siehe beispielsweise EP 1575600).

Die Konservierungsmittel können in die folgenden Klassen eingeteilt werden:
- Halogenorganischen Verbindungen, wie beispielsweise Triclosan (5-Chlor-2-(2,4-dichlorphenoxy)-phenol), lodopropynyl Butylcarbamate, Methylchloroisothiazolinone, Methyldibromo Glutaronitrile (MDGN), Chloracetamide, Dichlorphenyl-Imidazoldioxolan;
- Formaldehyd und Formaldehydabspalter, beispielsweise DMDM Hydantoin, Diazolidinyl Urea, Imidazolidinyl Urea, Sodium Hydroxymethylglycinate, Quaternium-15;
- halogenorganische Formaldehydabspalter, beispielsweise 5-Bromo-5-Nitro-1,3-Dioxane (Bronidox), 2-Bromo-2-Nitropropane-1,3-Diol (Bronopol);
- Parabene, beispielsweise Methylparaben, Ethylparaben, Propylparaben, Butylparaben, Isopropylparaben, Isobutylparaben.

Weitere Konservierungsstoffe, die in der Lebensmittelindustrie zugelassenen sind, haben Eingang in die Konservierung kosmetischer Zubereitungen gefunden, beispielsweise Sorbinsäure und Sorbate, Benzoesäure und Benzoate, PHB-Ester und Verbindungen.

Benzethonium Chlorid, mit dem internationalen Freinamen Benzyl-dimethyl-(4-{2-[4-(1,1,3,3-tetramethylbutyl)-phenoxy]-ethoxy}-ethyl)-ammoniumchlorid, ist ebenfalls ein bekanntes Konservierungsmittel, es handelt sich um eine quaternäre Ammoniumverbindung mit antimikrobiellen Eigenschaften. Diese Verbindung ist in Grapefruitkernextrakten in Konzentrationen von 7 bis 11 % enthalten.

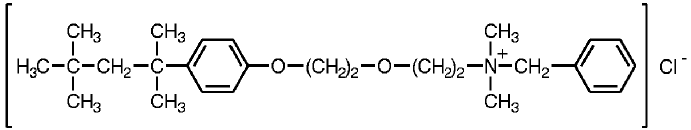

Diese Verbindung wird zur Konservierung von Arzneimitteln und Kosmetika eingesetzt.

In der Veröffentlichung US 2005/0238602 A1 wird die Verwendung von wasserlöslichen organischen Zinksalzen offenbart, die Irritationen entgegen wirken sollen, die durch Alkoholhaltige Desinfektionsmittel hervorgerufen werden. Diese Zinksalze werden in Zubereitungen eingearbeitet, die beispielsweise mit Benzethonium Chlorid konserviert werden.

Im Dokument WO 2001/007086 A1 wird offenbart, dass Benzethonium Chlorid in Kombination mit Phenoxyethanol oder Phenylethylalkohol eine gute konservierende Wirkung aufweist. Für die Kombination von Benzethonium Chlorid und Phenoxyethanol wird eine synergistische Wirkweise beschrieben.

Die Patentanmeldung US 2003/0196616 offenbart Hautpflegezusammensetzungen, deren Hauptwirkung die Vermeidung von Feuchtigkeitsverlusten der Haut ist. Die beschriebenen Zubereitungen enthalten Benzethonium Chlorid zur Konservierung. Siehe auch WO 2012/019789 A1.

Um die Wirkung von Konservierungsstoffen zu ergänzen oder zu verstärken, werden kosmetischen Zubereitungen Substanzen zugesetzt, die die Wirkung von Konservierungsmitten verstärken, so dass es möglich ist, die Konzentration des Konservierungsmittels abzusenken.

Ein Klasse von Verbindungen, die diese Funktion erfüllen können, sind Diole. Diole sind an sich bekannt. Es handelt sich um sind aliphatische und alicyclische Verbindungen mit zwei Hydroxylgruppen. Als Beispiele seien hier 1,2-Alkandiole, insbesondere 1,2-Pentandiol, 1,2-Hexandiol sowie auch 1,2-Octandiol, Ethylenglycol, Butandiole, 2,2-Dimethylpropan-1,3-diol und 2-Methyl-1,3-Propandiol genannt. Der Einsatz von Diolen in kosmetischen Zubereitungen ist an sich auch bekannt. Diole werden in vielen Fällen in der Kosmetik als Solubilisatoren eingesetzt.

Unter den Diolen gibt es Verbindungen, für die eine antimikrobielle Wirkung nachgewiesen wurde. Es handelt sich beispielsweise um 2-Brom-2-nitro-1,3-propandiol (Bronopol). Diese Verbindung wird auch den halogenorganischen Formaldehydabspaltern zugeordnet.

Im Dokument WO 2002/085324 A2, das angefeuchtete antimikrobielle Wischtücher offenbart, wird der Einsatz von Benzethonium Chlorid und Bronopol offenbart.

Ebenso ist beschrieben, dass Mischungen von bestimmten Diolen eine antimikrobielle Wirkung haben; dies wird offenbart in DE 20221386 U1. Die WO 2006/045743 A1 offenbart darüber hinaus, dass Mischungen bestimmter Diole (1,2-Hexandiol, 1,2 Octandiol, 1,2 Pentandiol, 1,2 Decanediol) mit bestimmten Konservierungsmitteln (Natrium Sorbat, Parabene, Iodopropynyl butylcarbamat (IPBC) zur Konservierung von Kosmetika, Pharmazeutika und Nahrungsmitteln verwendet werden können.

Dennoch besteht weiterhin der Bedarf, die Konservierung von kosmetischen Produkten zu optimieren. Besonders wünschenswert ist es, sichere Konservierungsmittel in geringen, aber wirksamen Mengen einsetzen zu können, die den Anforderungen, die an Kosmetika in Hinblick auf ihre Haltbarkeit gestellt werden, genügen zu können.

Die Aufgabe der vorliegenden Erfindung ist es daher, Wirkstoffkombinationen, die zur Konservierung kosmetischer Zubereitungen geeignet sind, zur Verfügung zu stellen. Diese Kombinationen sollen eine wirksame Konservierung erzielen, dabei aber sparsam im Einsatz sein. Ebenso sollen kosmetische Zubereitungen enthaltend derartige Wirkstoffkombinationen zur Konservierung zur Verfügung gestellt werden, die eine ausreichende mikrobiologische Langzeitstabilität aufweisen.

Überraschend wird diese Aufgabe gelöst durch eine Wirkstoffkombination aus Benzethonium Chlorid und den Verbindungen 2-Methyl-1,3-Propandiol und 1,2-Hexandiol, im folgenden auch allgemein als Diole bezeichnet. Erfindungsgemäss sind nur die Kombinationen, wie in den Ansprüchen definiert.

Offenbart sind auch kosmetische Zubereitungen, die eine Wirkstoffkombination aus Benzethonium Chlorid und Diolen enthalten, mit einem Gehalt an Benzethonium Chlorid von 0,01 bis 0,5 Gew. %, bevorzugt von 0,03 bis 0,3 Gew. %, ganz besonders bevorzugt von 0,05 bis 0,1 Gew. %, bezogen auf das Gesamtgewicht der Zubereitung und einem Gehalt an Diolen von 0,1 bis 5 Gew.-%, bezogen auf das Gesamtgewicht der Zubereitung. Bevorzugt ist zum einen die Kombination von Benzethomium Chlorid, insbesondere in den genannten Konzentrationsbereichen, mit 2-Methyl-1,3-Propandiol, wobei 2-Methyl-1,3-Propandiol in Konzentrationen von 0,1 bis 5 Gewichts. %, bevorzugt von 0,2 bis 4,5 Gew. %, besonders bevorzugt von 0,25 bis 4 Gew.-%, bezogen auf das Gesamtgewicht der Zubereitung, vorliegt. In einer weiteren bevorzugten Ausführungsform wird Benzethonium Chlorid, insbesondere in den genannten Konzentrationsbereichen, mit 1,2-Hexandiol kombiniert, wobei 1,2-Hexandiol in Gehalten von 0,1 bis 4 Gew. %, bevorzugt von 0,2 bis 1,0 Gew. %, besonders bevorzugt von 0,25 bis 0,75 Gew. %, bezogen auf das Gesamtgewicht der Zubereitung, vorliegt. Auch vorteilhaft sind Kombinationen von Benzethonium Chlorid, 2-Methyl-1,3-Propandiol und 1,2-Hexandiol, insbesondere in den jeweils genannten Konzentrationsbereichen. Im

Sinne der vorliegenden Erfindung sind die genannten Gehalte hinsichtlich der Diole als Gesamtmengen an Diolen zu verstehen. Sollte in einer erfindungsgemäßen Zubereitung ein bestimmtes Diol in der höchst möglichen Menge vorliegen, so dürfen keine weiteren Diole mehr zugesetzt werden.

Weiterhin erfindungsgemäß sind kosmetische Zubereitungen mit Wirkstoffkombinationen aus Benzethonium Chlorid und Diolen, in denen die Gewichtsverhältnisse von Benzethonium Chlorid zu Diolen aus dem Bereich 1:30 bis 1:3, bevorzugt aus dem Bereich 1:20 bis 1:4 ganz besonders bevorzugt aus dem Bereich 1:15 bis 1:5 gewählt werden.

Möglich sind auch Ausführungsformen, die zusätzlich Alkohole enthalten. Die Alkohole können ausgewählt werden aus der Gruppe der einwertigen Alkohole. Bevorzugt ist die Verwendung von Ethylalkohol oder Ethanol Alcohol denat. Wenn die genannten Alkohole den erfindungsgemäßen Zubereitungen zugesetzt werden, liegen die Alkohole in einer Konzentration von mehr als 1 Gew.-%, bezogen auf das Gesamtgewicht der Zubereitung, vor. Der Gehalt an Alkoholen sollte jedoch den Wert von 25 Gew.-%, bezogen auf das Gesamtgewicht der Zubereitung, nicht überschreiten.

Ebenso Gegenstand der vorliegenden Erfindung ist die Verwendung der Wirkstoffkombination aus Benzethonium Chlorid und Diolen zur Konservierung von Zubereitungen, bevorzugt von kosmetischen Zubereitungen, besonders bevorzugt von Emulsionen. Bevorzugt ist die Verwendung der Wirkstoffkombination aus Benzethonium Chlorid und 2-Methyl-1,3-Propandiol und/oder bestimmten 1,2-Alkandiolen, ganz besonders bevorzugt ist die Wirkstoffkombination aus Benzethonium Chlorid und 2-Methyl-1,3-Propandiol und/oder 1,2-Hexandiol.

Die erfindungsgemäßen Wirkstoffkombinationen von Benzethonium Chlorid und Diolen können in einer Vielzahl von kosmetischen, aber auch dermatologischen Zubereitungen mit unterschiedlichen galenischen Formen zum Einsatz kommen. Sie werden auf der Haut und/oder den Haaren angewandt im Sinne einer dermatologischen Behandlung oder einer Anwendung im Sinne der pflegenden und/oder reinigenden Kosmetik. Zur Ausführung von Schminkprodukten sind beispielsweise feste Stifte geeignet. Diese Stifte enthalten gemäß der Erfindung z. B. natürliche oder synthetische Wachse, Fettalkohole oder Fettsäureester. Bevorzugt werden Lippenpflegestifte sowie auch desodorierende Stifte ("Deo-Sticks").

Als Treibmittel für erfindungsgemäße, aus Aerosolbehältern versprühbare, kosmetische oder dermatologische Zubereitungen sind die üblichen bekannten leichtflüchtigen, verflüssigten Treibmittel, z. B. Kohlenwasserstoffe (Propan, Butan, Isobutan) geeignet, die allein oder in Mischung miteinander eingesetzt werden können. Auch Druckluft ist vorteilhaft zu verwenden.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, mindestens eine dialkylsubstituierte Carbonsäure im wässrigen Medium und Hilfsmittel, wie sie üblicherweise dafür verwendet werden.

Erfindungsgemäß vorteilhaft sind Emulsionen. Als Emulsion bezeichnet man ein System aus zwei nicht miteinander mischbaren Flüssigkeiten, wobei die eine Flüssigkeit in der anderen fein verteilt ist. Zur feinen Verteilung muss das System gerührt oder geschüttelt werden. Ein Emulgator verhindert die Entmischung kinetisch, denn Emulsionen sind in der Regel thermodynamisch nicht stabil. Häufig vorkommende Emulsionen sind solche aus einer Wasser- und einer Öl-Phase.

Bei W/O-Emulsionen ist die Ölphase durchgängig, die Wasserphase ist in kleinen Tröpfchen darin verteilt (Beispiel: Butter). Besteht die Ölphase im Wesentlichen aus Silikonöl, so spricht man von einer Wasser in Silikonemulsion (W/Si). Die erfindungsgemäßen W/O-Zubereitungen umfassen auch Wasser in Silikon Emulsionen. Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W-oder O/W/O-Emulsionen vorliegen. Solche Formulierungen können auch Mikroemulsionen, Stifte, Schäume (sog. Mousse), Feststoff-Emulsionen (d.h. Emulsionen, welche durch Feststoffe stabilisiert sind, z.B. Pickering-Emulsionen), sprühbare Emulsionen oder Hydrodispersionen sein.

Diese Art der Emulsion hat ihre Wurzeln bei den Herren Troplowitz, Lifschütz und Unna, welche die erste Wasser-in-ÖI-Emulsion zur Hautpflege zur Verfügung stellten. Lifschütz verwendete Wollwachs (Eucerit) als Emulgator. Beiersdorf gab dieser Creme den Namen Nivea (lat. nivis = Schnee).

Die Ölphase von Emulsionen kann vorteilhaft gewählt werden aus der folgenden Substanzgruppe:
- Mineralöle, Mineralwachse
- Öle, wie Triglyceride der Caprin- oder der Caprrylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie

Besonders vorteilhaft sind Mischungen aus C12-15-Alkylbenzoat und 2-Ethylhexylisostearat, Mischungen aus C12-15-Alkylbenzoat, 2-Ethylhexylisostearat und Isotridecylisononanoat.

Die wässrige Phase der Formulierungen im Sinne der vorliegenden Erfindung enthält gegebenenfalls vorteilhaft Alkohole oder Polyole niedriger C-Zahl, sowie deren Ether, vorzugsweise Ethanol, Isopropanol, Propylenglycol, Glycerin, Ethylenglykol, Ethylenglykolmonoethyl-oder -monobutylether, Propylenglykolmonomethyl-, -monoethyl- oder -monobutylether, Diethylenglykolmonomethyl- oder monoethylether und analoge Produkte, ferner Alkohole niedriger C-Zahl, z.B. Ethanol, Isopropanol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Xanthangummi und/oder Hydroxypropylmethylcellulose, besonders vorteilhaft aus der Gruppe der sogenannten-Carbopole, beispielsweise Carbopole der Typen 980, 981, 1382, 2984, 5984, jeweils einzeln oder in Kombination.

Weiterhin können die Wirkstoffkombinationen aus Benzethonium Chlorid und Diolen in ölfreien und/oder wässrigen/alkoholischen Lösungen eingesetzt werden. Auch in Gelen sind die erfindungsgemäßen Wirkstoffkombinationen aus Benzethonium Chlorid und Diolen vorteilhaft einzusetzen.

Gele gemäß der Erfindung enthalten üblicherweise Alkohole niedriger C-Zahl, z. B. Ethanol, Isopropanol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wässrig-alkoholischen oder alkoholischen Gelen vorzugweise ein Polyacrylat ist.

Verdickungsmittel sind Substanzen natürlichen oder synthetischen Ursprungs. Sie bestehen aus Mischungen von ähnlichen, aber nicht identischen Molekülen.

Viele verschiedene Verdickungsmittel sind bekannt: Alginat, Arabinoxylan, Carrageen, Carboxymethylcellulose, Cellulose, Gelatine, β-Glucan, Guar Gummi, Pektin, Stärke, Xanthan Gummi und andere.

Gummen werden aus Pflanzen- oder Baumsäften, die an der Luft erhärten und Harze bilden oder aus Extrakten aus Wasserpflanzen gewonnen. Hierzu zählen beispielsweise: Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin werden derivatisierte Gummen wie z.B. Hydroxypropyl Guar (Jaguar® HP 8) verwendet.

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Goodrich (Carbopol 980, 981, 1382, 5984, 2984, EDT 2001 oder Pemulen TR2).

Unter den Polymeren befinden sich z.B. Polyacrylamide (Seppigel 305), Polyvinylalkohole, PVP, PVP / VA Copolymere, Polyglycole.

Die erfindungsgemäßen Gele können beispielsweise im "rinse-off"-Bereich Anwendung finden, in Zubereitungen zur Haar- und Körperreinigung. Ebenso ist der Einsatz der erfindungsgemäßen Gele im "leave-on"-Bereich vorteilhaft. Unter Produkten des "rinse-off"-Bereichs sind Produkte zu verstehen, die auf die Haut aufgetragen werden, eine Wirkung erzielen und anschließend wieder abgespült werden. Produkte des "leave-on"-Bereichs sind zum Verbleib auf der Haut bestimmt.

Die erfindungsgemäßen Zubereitungen in Form von Emulsionen enthalten in der Regel Emulgatoren, die vorteilhaft gewählt werden können aus der Gruppe der nichtionischen, anionischen, kationischen oder amphoteren Emulgatoren.

Unter den nichtionischen Emulgatoren befinden sich
a) Partialfettsäureester und Fettsäureester mehrwertiger Alkohole und deren ethoxylierte Derivate (z. B. Glycerylmonostearate, Sorbitanstearate, Sucrosestearate)
b) ethoxylierte Fettalkohole und Fettsäuren
c) ethoxilierte Fettamine, Fettsäureamide, Fettsäurealkanolamide
d) Alkylphenolpolyglycolether (z. B. Triton X)
e) Zuckerderivate (Ester und/oder Ether von Glucose, Saccharose und anderen Zuckern ; z. B. Alkylpolyglycoside wie Polyglyceryl-3-Methylglucose-Distearat, Methylglucosesesquistearat)

Unter den anionischen Emulgatoren befinden sich
a) Seifen (z. B. Natriumstearat)
b) Fettalkoholsulfate
c) Mono-, Di-und Trialkylphosphosäureester und deren Ethoxylate.

Unter den kationischen Emulgatoren befinden sich
a) quaternäre Ammoniumverbindungen mit einem langkettigen aliphatischen Rest z. B. Distearytdimonium Chloride.

Unter den amphoteren Emulgatoren befinden sich
a) Alkylamininoalkancarbonsäuren
b) Betaine, Sulfobetaine
c) Imidazolinderivate

Weiterhin gibt es natürlich vorkommende Emulgatoren, zu denen Bienenwachs, Woll- wachs, Lecithin und Sterole gehören.

Emulgatoren mit HLB-Werten von 6-8 sind im Allgemeinen W/O-Emulgatoren, solche mit HLB-Werten von 8-18 sind im Allgemeinen O/W-Emulgatoren. Hydrophile Emulgatoren (mit hohen HLB-Werten) sind in der Regel O/W-Emulgatoren. Demgemäß sind hydrophobe oder lipophile Emulgatoren (mit niedrigen HLB-Werten) in der Regel W/O-Emulgatoren.

O/W-Emulgatoren können beispielsweise vorteilhaft gewählt werden aus der Gruppe der polyethoxylierten bzw. polypropoxylierten bzw. polyethoxylierten und polypropoxylierten Produkte, z. B.:
- der Fettalkoholethoxylate
- der ethoxylierten Wollwächsalkohole,
- der Polyethylenglycolether der allgemeinen Formel R-O- (-CH2-CH2-0-) n-R', - der Fettsäureethoxylate der allgemeinen Formel R-COO- (-CH2-CH2-O-)n-H,
- der veretherten Fettsäureethoxylate der allgemeinen Formel R-COO-(-CH2-CH2-O-)n-R',
- der veresterten Fettsäureethoxylate der allgemeinen Formel R-COO-(-CH2-CH2-O-)n-C (O)-R',
- der Polyethylenglycolglycerinfettsäureester der ethoxylierten Sorbitanester der Cholesterinethoxylate der ethoxylierten Triglyceride der Alkylethercarbonsäuren der allgemeinen Formel R-O-(-CH2-CH2-O-)n-CH2-COOH nd n eine Zahl von 5 bis 30 darstellen,
- der Polyoxyethylensorbitolfettsäureester,
- der Alkylethersulfate der allgemeinen Formel R-O-(-CH2-CH2-O-)n-SO3-H der Fettalkoholpropoxylate der allgemeinen Formel R-O-(-CH2-CH(CH3)-O-)n-H, der Polypropylenglycolether der allgemeinen Formel R-O-(-CH2-CH(CH2)-O-)n-R',
- der propoxylierten Wollwachsalkohole,
   der veretherten Fettsäurepropoxylate R-COO-(-CH2-CH(CH3)-O-)n-R',
   der veresterten Fettsäurepropoxylate der allgemeinen Formel R-COO-(-CH2-CH(CH3)-O-)n-C(O)-R',
   der Fettsäurepropoxylate der allgemeinen Formel R-COO-(-CH2-CH(CH3)-O-)n-H, der Polypropylenglycolglycerinfettsäureester der propoxylierten Sorbitanester der Cholesterinpropoxylate der propoxylierten Triglyceride der Alkylethercarbonsäuren der allgemeinen Formel R-O- (-CH2-CH (CH3) 0-) n-CH2-COOH
   der Alkylethersulfate bzw. die diesen Sulfaten zugrundeliegenden Säuren der allgemeinen Formel R-O-(-CH2-CH(CH3)-O-)n-SO3-H der Fettalkoholethoxylate/propoxylate der allgemeinen Formel R-O-Xn-Ym-H, der Polypropylenglycolether
   der allgemeinen Formel R-O-Xn-Ym-R',
   der veretherten Fettsäurepropoxylate der allgemeinen Formel R-COO-Xn-Ym-R',
- der Fettsäureethoxylate/propoxylate der allgemeinen Formel R-COO-Xn-Ym-H,

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11-18, ganz besonders vorteilhaft mit mit HLB-Werten von 14,5-15, 5, sofern die O/W-Emulgatoren gesättigte Reste R und R'aufweisen. Weisen die O/W-Emulgatoren ungesättigte Reste R und/oder R'auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylal- kohole, Cetylalkohole, Cetylstearylalkohole (Cetearylalkohole) zu wählen. Insbesondere bevorzugt sind: Polyethylenglycol (13) stearylether (Steareth-13), Polyethylenglycol (14) stearylether (Stea- reth-14), Polyethylenglycol (15) stearylether (Steareth-15), Polyethylenglycol (16) stearyl- ether (Steareth-16), Polyethylenglycol (17) stearylether (Steareth-17), Polyethylenglycol- (18) stearylether (Steareth-18), Polyethylenglycol (19) stearylether (Steareth-19), Polyethy- lenglycol (20) stearylether (Steareth-20), Polyethylenglycol (12) isostearylether (Isosteareth-12), Polyethylenglycol (13) isostearyl- ether (Isosteareth-13), Polyethylenglycol (14) isostearylether (Isosteareth-14), Polyethylen- glycol (15) isostearylether (Isosteareth-15), Polyethylenglycol (16) isostearylether (Isostea- reth-16), Polyethylenglycol (17) isostearylether (Isosteareth-17), Polyethylenglycol- (18) isostearylether (Isosteareth-1), Polyethylenglycol (19) isostearylether (Isosteareth-19-), Polyethylenglycol (20) isostearylether (Isosteareth-20), Polyethylenglycol (13) cetylether (Ceteth-13), Polyethylenglycol (14) cetylether (Ceteth-14), Polyethylenglycol (15) cetylether (Ceteth-15), Polyethylenglycol (16) cetylether (Ceteth-16), Polyethylenglycol (17) cetylether (Ceteth-17), Polyethylenglycol (18) cetylether (Ceteth-18), Polyethylenglycol (19) cetylether (Ceteth-19), Polyethylenglycol (20) cetylether (Ceteth-20), Polyethylenglycol (13) isocetylether (Isoceteth-13), Polyethylenglycol (14) isocetylether (Iso- ceteth-14), Polyethylenglycol (15) isocetylether (Isoceteth-15), Polyethylenglycol (16) iso- cetylether (Isoceteth-16), Polyethylenglycol (17) isocetylether (Isoceteth-17), Polyethylenglycol (18) isocetylether (Isoceteth-18), Polyethylenglycol (19) isocetylether (Isoceteth-19), Polyethylenglycol (20) isocetylether (Isoceteth-20), Polyethylenglycol (12) oleylether (Oleth-12), Polyethylenglycol (13) oleylether (Oleth-13), Polyethylenglycol (14) oleylether (Oleth-14), Polyethylenglycol (15) oleylether (Oleth-15), Polyethylenglycol (12) laurylether (Laureth-12), Polyethylenglycol (12) isolaurylether (Isolaureth-12).

Polyethylenglycol (13) cetylstearylether (Ceteareth-13), Polyethylenglycol (14) cetylstearylether (Ceteareth-14), Polyethylenglycol (15) cetylstearylether (Ceteareth-15), Polyethylenglycol (16) cetylstearylether (Ceteareth-16), Polyethylenglycol (17) cetylstearylether (Ceteareth-17), Polyethylenglycol (18) cetylstearylether (Ceteareth-18), Polyethylenglycol (19) - cetylstearylether (Ceteareth-19), Polyethylenglycol (20) cetyistearylether (Ceteareth-20), Es ist ferner von Vorteil, die Fettsäureethoxylate aus folgender Gruppe zu wählen : Polyethylenglycol (20) stearat, Polyethylenglycol (21) stearat, Polyethylenglycol (22) stearat, Polyethylenglycol (23) stearat, Polyethylenglycol (24) stearat, Polyethylenglycol (25) stearat, Polyethylenglycoi (12) isostearat, Polyethylenglycol (13) isostearat, Polyethylenglycol (14) isostearat, Polyethylenglycol (15) isostearat, Polyethylenglycol (16) isostearat, Polyethylenglycol (17) isostearat, Polyethylenglycol (18) isostearat, Polyethylenglycol (19) isostearat, Polyethylenglycol (20) isostearat, Polyethylenglycol (21) isostearat, Polyethylenglycol- (22) isostearat, Polyethylenglycol (23) isostearat, Polyethylenglycol (24) isostearat, Polyethylenglycol (25) isostearat, Polyethylenglycol (12) oleat, Polyethylenglycol (13) oleat, Polyethylenglycol (14) oleat, Polyethylenglycol (15) oleat, Polyethylenglycol (16) oleat, Polyethylenglycol (17) oleat, Polyethy- lenglycol (18) oleat, Polyethylenglycol (19) oleat, Polyethylenglycol (20) oleat Als ethoxylierte Alkylethercarbonsäure bzw. deren Salz kann vorteilhaft das Natriumlau- reth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol (30) Cholesterylether verwendet werden. Auch Polyethylenglycol (25) Sojasterol hat sich bewährt.

Als ethoxylierte Triglyceride können vorteilhaft die Polyethylenglycol (60) Evening Primrose Glycerides verwendet werden (Evening Primrose = Nachtkerze) Weiterhin ist von Vorteil, die Polyethylenglycolglycerinfettsäureester aus der Gruppe Polyethylenglycol (20) glyceryllaurat, Polyethylenglycol (21) glyceryllaurat, Polyethylenglycol- (22) glyceryllaurat, Polyethylenglycol (23) glyceryllaurat, Polyethylenglycol (6) glycerylcaprat/caprinat, Polyethylenglycol (20) glyceryloleat, Polyethylenglycol (20) glycerylisostea- rat, Polyethylenglycol (18) glyceryloleat/cocoat zu wählen.

Es ist ebenfalls günstig, die Sorbitanester aus der Gruppe Polyethylenglycol (20) sorbitanmonolaurat, Polyethylenglycol (20) sorbitanmonostearat, Polyethylenglycol (20) sorbitanmonoisostearat, Polyethylenglycol (20) sorbitanmonopalmitat, Polyethylenglycol (20) sorbitanmonooleat zu wählen.

Als vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atomen, Diglycerinester gesättigter und/oder ungesättigter,
verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglyceryl- monoisostearat, Propylenglycolmonostearat, Propylenglycolmonoisostearat, Propylengly- colmonocaprylat, Propylenglycolmonolaurat, Sorbitanmonoisostearat, Sorbitanmonolau- rat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol (2) stearylether (Steareth-2), Glycerylmonolaurat, Glyce- rylmonocaprinat, Glycerylmonocaprylat.

Ein erfindungsgemäß bevorzugter Emulgator ist das Glycerylstearatcitrat. Dieser ist beispielsweise unter den Produktbezeichnungen "IMWITOR 370" von der Firma Cremer Oleo und "Axol C 62" von der Firma Evonik erhältlich.

Erfindungsgemäß können auch Siliconemulgatoren verwendet werden, die grenzflächenaktive Substanzen sind und aus der Gruppe der Alkylmethiconcopolyole und/oder Alkyl-Dimethiconcopolyole gewählt werden können, insbesondere aus der Gruppe der Verbindungen, welche gekennzeichnet sind durch die folgende chemische Struktur: 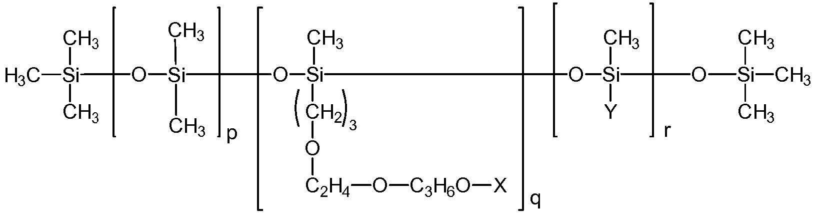 bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Beispielhaft seien Dimethiconcopolyole genannt, welche von der Firma Evonik Industries unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden. Weiterhin können Substanzen wie das Cetyl Dimethiconcopolyol, welches auch von der Firma Evonik Industries unter der Warenbezeichnung ABIL® EM 90 verkauft wird oder Cyclomethicon Dimethiconcopolyol, welches gleichfalls von der Firma Evonik Industries unter der Warenbezeichnung ABIL® EM 97 verkauft wird, genannt werden. Ebenso einsetzbar ist der Emulgator Laurylmethiconcopolyol, welcher unter der Warenbezeichnung Dow Corning® 5200 Formulation Aid von der Gesellschaft Dow Corning Ltd. erhältlich ist.

Enthalten erfindungsgemäße Ausführungsformen Emulgatoren, so sind die Emulgatoren mit einem Gesamtgehalt von 0,1 bis 7,5 Gew.-%, bezogen auf das Gesamtgewicht der Zubereitung vorhanden.

Tenside können ebenfalls in den erfindungsgemäßen Zubereitungen Verwendung finden. Die Tenside können dann anionische und/oder amphotere und/oder kationische und/oder nichtionische Tenside sein und in Gehalten von 0,5 bis 20 Gew.-%, bezogen auf das Gesamtgewicht der Zubereitung vorliegen.

Vorteilhaft sind auch kosmetische und dermatologische Zubereitungen, die in der Form eines Sonnenschutzmittels vorliegen. Vorteilhaft enthalten diese mindestens einen UVA-Filter und/oder mindestens einen UVB-Filter und/oder mindestens ein anorganisches Pigment. Die Gesamtmenge der Filtersubstanzen beträgt 3 Gew.-% bis 40 Gew.-%, vorzugsweise 5 bis 35 Gew.-%, insbesondere 10 bis 30 Gew.-%, bezogen auf das Gesamtgewicht der Zubereitung, um kosmetische Zubereitungen zur Verfügung zu stellen, die die Haut vor dem gesamten Bereich der ultravioletten Strahlung schützen.

Die kosmetischen Zubereitungen gemäß der Erfindung können kosmetische Hilfsstoffe enthalten, wie sie üblicherweise in solchen Zubereitungen verwendet werden, z. B. Antioxidantien, Parfüme, Mittel zum Verhindern des Schäumens, Farbstoffe, Pigmente, die eine färbende Wirkung haben, weichmachende Substanzen, anfeuchtende und/oder feuchthaltende Substanzen, rückfettende Agentien, Fette, Öle, Wachse und deren toxikologisch verträgliche Ether und Ester, Stabilisatoren, pH-Wert-Regulatoren, Konsistenzgeber, Desodorantien, Antistatika, Komplexierungs- und Sequestrierungsagentien, Perlglanzagentien, Polymere, Elektrolyte, weitere organische Lösungsmittel, Silikonderivate, Pflanzenextrakte, Vitamine und/oder andere Wirkstoffe oder andere übliche Bestandteile einer kosmetischen Formulierung. Auch weitere Lösungsvermittler, z.B. zur Einarbeitung hydrophober Komponenten wie z.B. von Parfümzubereitungen können enthalten sein.

Die kosmetischen Zubereitungen können auch Wirkstoffe und Hilfsstoffe enthalten, wie sie üblicherweise für Zubereitungen zur Haarpflege und Haarbehandlung verwendet werden, wie beispielsweise spezielle in der Haarkosmetik übliche kationische oberflächenaktiven Substanzen, Substanzen zum Verhindern des Schäumens, Farbstoffe oder Pigmente, deren Aufgabe es ist, die Haare oder die Zubereitung selbst zu färben, Zubereitungen gegen das Fetten der Haare, Substanzen gegen Schuppen, Antimykotika speziell geeignet für Haarzubereitungen.

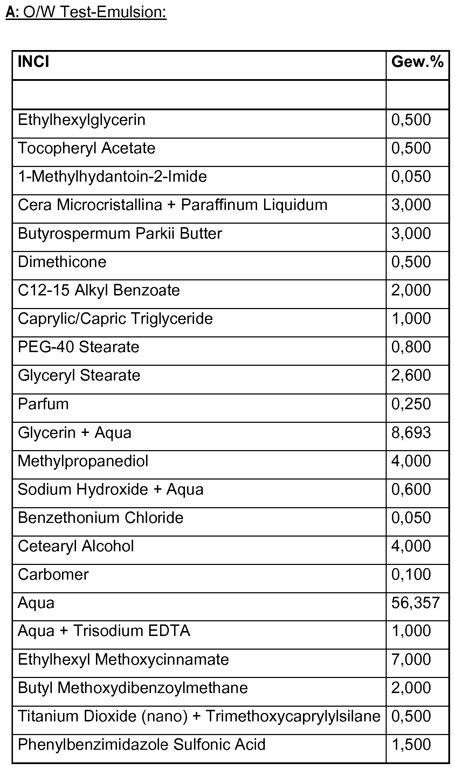

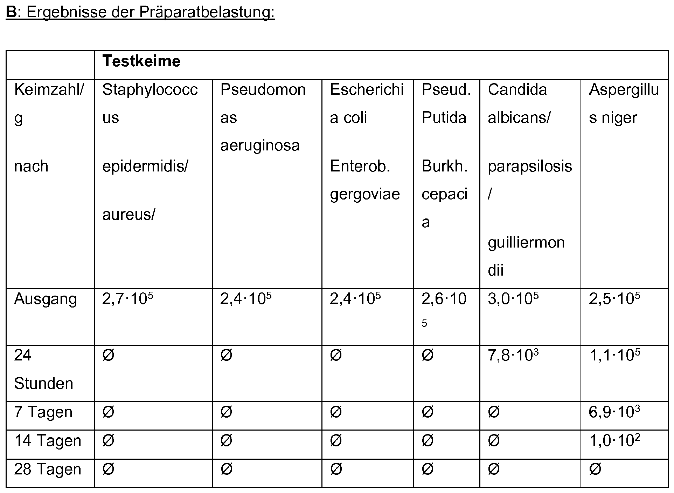

Um die antimikrobielle Wirksamkeit der erfindungsgemäßen Kombination zu belegen wurde eine Konservierungsmittelprüfung in Form einer Präparatbelastung durchgeführt. Dazu wurde eine O/W Test-Emulsion mit verschiedenen Keimen in Kontakt gebracht. Die Absterbekinetik von ausgewählten Keime wurde untersucht, siehe dazu Abbildung 1.

Es wird deutlich, dass die erfindungsgemäße Kombination von 4% 2-Methyl-1,3-Propandiol und 0,05 % Benzethonium Chlorid sehr gute Wirksamkeit gegen Bakterien zeigt. Auch Candida-Species werden sehr gut beherrscht. Lediglich bei Aspergillus niger erfolgte keine so rasche Keimabtötung, die Ergebnisse zeigen aber, dass auch dieser Keim abgetötet wird.

Die erfindungsgemäßen kosmetischen Zubereitungen sind überaus haut- und anwendungsfreundliche Zusammensetzungen, die ein geringes Hautirritationspotential aufweisen. Ebenso können die erfindungsgemäßen Wirkstoffkombinationen zur Konservierung in dermatologische Zubereitungen eingearbeitet werden.

Die folgenden Beispiele sollen die vorliegende Erfindung verdeutlichen. Die Angaben beziehen sich stets auf Gew.-%, sofern nicht andere Angaben gemacht werden.

### Beispiele:

| 0/W Emulsion | | 0/W Emulsion | | 0/W Emulsion | | 2-Phasen Spray | |
|---|---|---|---|---|---|---|---|
| PEG 40 Stearate | 0,8 | Potassium Cetyl Phosphate + Hydrogenated Palm Glycerides | 1,0 | Glyceryl Stearate Citrate | 1,5 | Isopropyl Palmitate | 10,0 |
| Cetylalkohol | 0,8 | Vaseline | 3,0 | Cetylalkohol | 0,5 | C12-15 Alkyl Benzoate | 10,0 |
| Sheabutter | 3,0 | Ocytyldodecanol | 5,0 | Ocytyldodecanol | 3,0 | | |
| Dimethicone | 0,5 | Cyclomethicone | 1,0 | Dimethicone | 2,0 | Dimethicone | 1,0 |
| Octyl Methoxycinnamate | 7,0 | Octyl Methoxycinnamate | 5,0 | Myristyl Myristate | 1,0 | | |
| Butyl Methoxy-benzoylmethan | 2,0 | Octocrylene | 2,0 | Dicaprylyl Carbonate | 5,0 | | |
| C12-15 Alkyl Benzoate | 2,0 | Neutralfett | 3,0 | C12-15 Alkyl Benzoate | 2,0 | | |
| Glyceryl Stearate | 2,5 | Glyceryl Stearate | 2,5 | Tapioka Starch | 2,0 | | |
| Titandioxide | 0,5 | | | | | | |
| Caprylic Capric Triglyceride | 1,0 | | | | | | |
| Carragenan | 0,2 | Carbomer | 0,15 | Carbomer | 0,4 | | |
| Carbomer | 0,1 | | | Ammonium Acryloyldimethyltaurate/VP Copolymer | 0,3 | | |
| Glycerin | 9,0 | Glycerin: | 8,0 | Glycerin | 8,0 | Glycerin | 12,0 |
| Natriumhydroxid | 0,13 | Natriumhydroxid | 0,11 | Natriumhydroxid | 0,3 | | |
| Fragrance | 0,3 | Fragrance | 0,3 | Fragrance | 0,05 | Fragrance | 0,2 |
| Methylpropanediol | 4,0 | Methylpropanediol | 2,0 | Methylpropanediol: | 2,0 | | |
| | | 1,2, Hexanediol | 0,5 | | | 1,2-Hexandiol | 1,0 |
| Benzethoniumchlorid | 0,05 | Benzethoniumchlorid | 0,05 | Benzethoniumchlorid | 0,05 | Benzethoniumchlorid | 0,04 |
| Wasser | Ad 100 | Wasser | Ad 100 | Wasser | Ad 100 | Wasser | Ad 100 |

## Patentansprüche

1. Wirkstoffkombination bestehend aus Benzethonium Chlorid und einem oder mehreren Diol(en), **dadurch gekennzeichnet, dass** das oder die Diol(e) 2-Methyl-1,3-Propandiol und/oder 1,2-Hexandiol ist/sind.

2. Kosmetische Zubereitung, enthaltend eine Wirkstoffkombination gemäß Anspruch 1, **dadurch gekennzeichnet, dass**
Benzethonium Chlorid mit einem Gehalt von 0,01 bis 0,5 Gew. %, bevorzugt von 0,03 bis 0,3 Gew. %, ganz besonders bevorzugt von 0,05 bis 0,1 Gew. %, bezogen auf das Gesamtgewicht der Zubereitung und dass
das Diol 1,2-Hexandiol ist und mit einem Gehalt von 0,1 bis 4 Gew. %, bevorzugt 0,2 bis 1,0 Gew. %, besonders bevorzugt 0,25 bis 0,75 Gew. %, bezogen auf das Gesamtgewicht der Zubereitung, enthalten ist oder dass
die Diole 2-Methyl-1,3-Propandiol und 1,2-Hexandiol enthalten sind, wobei 1,2-Hexandiol mit einem Gehalt von 0,1 bis 4 Gew. %, bevorzugt 0,2 bis 1,0 Gew. %, besonders bevorzugt 0,25 bis 0,75 Gew. %, bezogen auf das Gesamtgewicht der Zubereitung vorliegt und wobei 2-Methyl-1,3-Propandiol mit einem Gehalt von 0,1 bis 5 Gew. %, bevorzugt 0,2 bis 4,5 Gew. %, besonders bevorzugt 0,25 bis 4 Gew. % vorliegt, bezogen auf das Gesamtgewicht der Zubereitung.

3. Kosmetische Zubereitung gemäß Anspruch 2 **dadurch gekennzeichnet, dass** zusätzlich Alkohole, insbesondere Ethylalkohol oder Ethanol Alcohol denat. mit einem Gehalt von mehr als 1 Gew. %, bezogen auf das Gesamtgewicht der Zubereitung, enthalten sind.

4. Kosmetische Zubereitung gemäß wenigstens einem der Ansprüche 2 und/oder 3, **dadurch gekennzeichnet, dass** die Zubereitung in Form einer Emulsion vorliegt.

5. Kosmetische Zubereitung gemäß Anspruch 4, **dadurch gekennzeichnet, dass** die Zubereitung in Form einer O/W-Emulsion vorliegt.

6. Kosmetische Zubereitung gemäß wenigstens einem der Ansprüche 2 und/oder 3 **dadurch gekennzeichnet, dass** die Zubereitung in Form eines 2-Phasen-Sprays vorliegt.

7. Verwendung der Wirkstoffkombination gemäß Anspruch 1 zur Konservierung von Zubereitungen, bevorzugt von kosmetischen Zubereitungen, besonders bevorzugt von Emulsionen.

8. Verwendung nach Anspruch 7, wobei
Benzethonium Chlorid mit einem Gehalt von 0,01 bis 0,5 Gew. %, bevorzugt von 0,03 bis 0,3 Gew. %, ganz besonders bevorzugt von 0,05 bis 0,1 Gew. %, bezogen auf das Gesamtgewicht der Zubereitung und dass das Diol 1,2-Hexandiol ist und mit einem Gehalt von 0,1 bis 4 Gew. %, bevorzugt 0,2 bis 1,0 Gew. %, besonders bevorzugt 0,25 bis 0,75 Gew. %, bezogen auf das Gesamtgewicht der Zubereitung, enthalten ist oder dass
die Diole 2-Methyl-1,3-Propandiol und 1,2-Hexandiol enthalten sind, wobei 1,2-Hexandiol mit einem Gehalt von 0,1 bis 4 Gew. %, bevorzugt 0,2 bis 1,0 Gew. %, besonders bevorzugt 0,25 bis 0,75 Gew. %, bezogen auf das Gesamtgewicht der Zubereitung vorliegt und wobei 2-Methyl-1,3-Propandiol mit einem Gehalt von 0,1 bis 5 Gew. %, bevorzugt 0,2 bis 4,5 Gew. %, besonders bevorzugt 0,25 bis 4 Gew. % vorliegt, bezogen auf das Gesamtgewicht der Zubereitung.

## Claims

1. Active ingredient combination consisting of benzethonium chloride and one or more diol(s), **characterized in that** the diol(s) is/are 2-methyl-1,3-propanediol and/or 1,2-hexanediol.

2. Cosmetic preparation comprising an active ingredient combination according to Claim 1, **characterized in that**
benzethonium chloride is present at a content of 0.01 to 0.5% by weight, preferably 0.03 to 0.3% by weight, especially preferably 0.05 to 0.1% by weight, based on the total weight of the preparation and that
the diol is 1,2-hexanediol and is present at a content of 0.1 to 4% by weight, preferably 0.2 to 1.0% by weight, particularly preferably 0.25 to 0.75% by weight, based on the total weight of the preparation or that
the diols 2-methyl-1,3-propanediol and 1,2-hexanediol are present, wherein 1,2-hexanediol is present at a content of 0.1 to 4% by weight, preferably 0.2 to 1.0% by weight, particularly preferably 0.25 to 0.75% by weight, based on the total weight of the preparation and wherein 2-methyl-1,3-propanediol is present at a content of 0.1 to 5% by weight, preferably 0.2 to 4.5% by weight, particularly preferably 0.25 to 4% by weight, based on the total weight of the preparation.

3. Cosmetic preparation according to Claim 2, **characterized in that** additional alcohols are present, particularly ethyl alcohol or ethanol denatured alcohol at a content of more than 1% by weight, based on the total weight of the preparation.

4. Cosmetic preparation according to at least one of Claims 2 and/or 3, **characterized in that** the preparation is in the form of an emulsion.

5. Cosmetic preparation according to Claim 4, **characterized in that** the preparation is in the form of an O/W emulsion.

6. Cosmetic preparation according to at least one of Claims 2 and/or 3, **characterized in that** the preparation is in the form of a 2-phase spray.

7. Use according to Claim 1 for preserving preparations, preferably cosmetic preparations, particularly preferably emulsions.

8. Use according to Claim 7, wherein
benzethonium chloride is present at a content of 0.01 to 0.5% by weight, preferably 0.03 to 0.3% by weight, especially preferably 0.05 to 0.1% by weight, based on the total weight of the preparation and that
the diol is 1,2-hexanediol and is present at a content of 0.1 to 4% by weight, preferably 0.2 to 1.0% by weight, particularly preferably 0.25 to 0.75% by weight, based on the total weight of the preparation or that
the diols 2-methyl-1,3-propanediol and 1,2-hexanediol are present, wherein 1,2-hexanediol is present at a content of 0.1 to 4% by weight, preferably 0.2 to 1.0% by weight, particularly preferably 0.25 to 0.75% by weight, based on the total weight of the preparation and wherein 2-methyl-1,3-propanediol is present at a content of 0.1 to 5% by weight, preferably 0.2 to 4.5% by weight, particularly preferably 0.25 to 4% by weight, based on the total weight of the preparation.

## Revendications

1. Combinaison de principe actif constituée de chlorure de benzéthonium et d'un ou plusieurs diol(s), **caractérisée en ce que** le ou les diol(s) est/sont le 2-méthyl-1,3-propanediol et/ou le 1,2-hexanediol.

2. Préparation cosmétique, contenant une combinaison de principe actif selon la revendication 1, **caractérisée en ce que**
le chlorure de benzéthonium est contenu en une teneur de 0,01 à 0,5 % en poids, préférablement de 0,03 à 0,3 % en poids, tout particulièrement préférablement de 0,05 à 0,1 % en poids, par rapport au poids total de la préparation et **en ce que** le diol est le 1,2-hexanediol et est contenu en une teneur de 0,1 à 4 % en poids, préférablement 0,2 à 1,0 % en poids, particulièrement préférablement 0,25 à 0,75 % en poids, par rapport au poids total de la préparation, ou **en ce que**
les diols 2-méthyl-1,3-propanediol et 1,2-hexanediol sont contenus, le 1,2-hexanediol étant présent en une teneur de 0,1 à 4 % en poids, préférablement 0,2 à 1,0 % en poids, particulièrement préférablement 0,25 à 0,75 % en poids, par rapport au poids total de la préparation et le 2-méthyl-1,3-propanediol étant présent en une teneur de 0,1 à 5 % en poids, préférablement 0,2 à 4,5 % en poids, particulièrement préférablement 0,25 à 4 % en poids, par rapport au poids total de la préparation.

3. Préparation cosmétique selon la revendication 2 **caractérisée en ce que** des alcools, en particulier de l'alcool éthylique ou de l'alcool d'éthanol dénaturé, sont en outre contenus en une teneur de plus de 1 % en poids, par rapport au poids total de la préparation.

4. Préparation cosmétique selon au moins l'une des revendications 2 et 3, **caractérisée en ce que** la préparation est présente sous forme d'une émulsion.

5. Préparation cosmétique selon la revendication 4, **caractérisée en ce que** la préparation est présente sous forme d'une émulsion huile/eau.

6. Préparation cosmétique selon au moins l'une des revendications 2 et 3, **caractérisée en ce que** la préparation est présente sous forme d'un spray à 2 phases.

7. Utilisation de la combinaison de principe actif selon la revendication 1 pour la conservation de préparations, préférablement de préparations cosmétiques, particulièrement préférablement d'émulsions.

8. Utilisation selon la revendication 7,
le chlorure de benzéthonium étant contenu en une teneur de 0,01 à 0,5 % en poids, préférablement de 0,03 à 0,3 % en poids, tout particulièrement préférablement de 0,05 à 0,1 % en poids, par rapport au poids total de la préparation et
le diol étant le 1,2-hexanediol et étant contenu en une teneur de 0,1 à 4 % en poids, préférablement 0,2 à 1,0 % en poids, particulièrement préférablement 0,25 à 0,75 % en poids, par rapport au poids total de la préparation, ou
les diols 2-méthyl-1,3-propanediol et 1,2-hexanediol étant contenus, le 1,2-hexanediol étant présent en une teneur de 0,1 à 4 % en poids, préférablement 0,2 à 1,0 % en poids, particulièrement préférablement 0,25 à 0,75 % en poids, par rapport au poids total de la préparation et le 2-méthyl-1,3-propanediol étant présent en une teneur de 0,1 à 5 % en poids, préférablement 0,2 à 4,5 % en poids, particulièrement préférablement 0,25 à 4 % en poids, par rapport au poids total de la préparation.
